(19) Europäisches Patentamt
European Patent Office
Office européen des brevets

(11) EP 1 347 013 A2

(12) **EUROPÄISCHE PATENTANMELDUNG**

(43) Veröffentlichungstag: **24.09.2003 Patentblatt 2003/39**

(21) Anmeldenummer: **03005897.8**

(22) Anmeldetag: **17.03.2003**

(51) Int Cl.[7]: **C08L 51/00**, C08F 291/00, C08F 290/06, C08F 265/00, C08F 265/10, A61K 7/00

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **23.03.2002 DE 10213142**

(71) Anmelder: **Clariant GmbH**
**65929 Frankfurt am Main (DE)**

(72) Erfinder:
- **Loeffler, Matthias Dr.**
  **65527 Niedernhausen (DE)**
- **Morschhaeser, Roman Dr.**
  **55122 Mainz (DE)**

(74) Vertreter: **Paczkowski, Marcus, Dr. et al**
**Clariant Service GmbH**
**Patente, Marken, Lizenzen**
**Am Unisys-Park 1**
**65843 Sulzbach (DE)**

(54) **Stabile Dispersionskonzentrate**

(57) Gegenstand der Erfindung sind Dispersionskonzentrate, enthaltend mindestens ein Copolymer, erhältlich durch radikalische Copolymerisation von

A) Acryloyldimethyltaurinsäure und/oder Acryloyldimethyltauraten,
B) gegebenenfalls einem oder mehreren weiteren olefinisch ungesättigten, nicht kationischen, Comonomeren,
C) gegebenenfalls einem oder mehreren olefinisch ungesättigten, kationischen Comonomeren,
D) gegebenenfalls einer oder mehreren siliziumhaltigen Komponente(n),
E) gegebenenfalls einer oder mehreren fluorhaltigen Komponente(n),
F) gegebenenfalls einem oder mehreren Makromonomeren,
G) wobei die Copolymerisation gegebenenfalls in Gegenwart mindestens eines polymeren Additivs erfolgt,

mit der Maßgabe, dass die Komponente A) mit mindestens einer Komponente ausgewählt aus einer der Gruppen D) bis G) copolymerisiert wird.

EP 1 347 013 A2

## Beschreibung

Die vorliegende Erfindung betrifft Dispersionskonzentrate, enthaltend Copolymere auf Basis von Acryloyidimethyltaurinsäure bzw. deren Salze (AMPS), erhältlich durch Copolymerisation von AMPS mit einem oder mehreren olefinischen Comonomer/en und Komponenten mit funktionellen Gruppen.

In der Anmeldung PCT/EP 01/13860 wird eine neue Klasse von Polymeren auf Basis von Acryloyldimethyltaurinsäure bzw. deren Salze beschrieben. Diese Polymere decken breite anwendungstechnische Eigenschaften ab und können als Verdicker, Konsistenzgeber, Emulgator, Dispergator, Gleitmittel, Conditioner und/oder Stabilisator in kosmetischen, dermatologischen und pharmazeutischen Mitteln eingesetzt werden.

Die bevorzugt durch Fällungspolymerisation hergestellten Copolymere auf Basis von AMPS entsprechend dem Stand der Technik sind pulverförmige Substanzen mit dadurch resultierenden anwendungstechnischen Nachteilen. Neben einer Staubexplosionsgefahr kann der Staub Gefahren bei Inhalation bergen, ferner ist die Lagerstabilität der Pulver durch Hygroskopie beeinträchtigt.

Bei Verarbeitung bzw. Verwendung der pulverförmigen Produkte ist der Lösevorgang (bevorzugt werden die Polymere in wässrige Medien eingearbeitet) meistens sehr zeitaufwendig. Der Lösevorgang der pulverförmigen Produkte kann, je nach Ansatzgröße, eine Stunde und mehr betragen. Zudem wird häufig eine unvollständige Lösung/Aufquellung der pulverförmigen Produkte beobachtet, was zu einer Qualitäts- und Stabilitätsverminderung der Endformulierung führt (Klumpenund Quaddelbildung). Des weiteren sind bei der Verarbeitung bzw. Verwendung der pulverförmigen Produkte i.a. besondere Rühr- und Dispergiervorrichtungen erforderlich, um die AMPS-Polymere in den Mitteln zu lösen, bzw. zu suspendieren.

Zielsetzung war, flüssige Formen der pulverförmigen Polymere auf Basis von Acryloyldimethyltaurinsäure bzw. deren Salze, bevorzugt hergestellt durch Fällungspolymerisation (AMPS) zu finden. Bevorzugt sind dabei Dispersionen der Polymere in einer flüssigen Matrix enthaltend Öl, Emulgator, Dispergator und/oder Wasser. Bevorzugt sind dabei flüssig-disperse Formen mit möglichst hohem Polymeranteil, niedriger Viskosität bei gleichzeitig hoher Stabilität der Dispersion. Die verwendeten Öl- und Emulgator/Dispergatoranteile sind bevorzugt kosmetisch und pharmazeutisch akzeptable Rohstoffe.

Überraschenderweise wurde gefunden, dass sich AMPS-Copolymere hervorragend eignen zur Herstellung von Dispersionskonzentraten.

Gegenstand der Erfindung sind Dispersionskonzentrate, enthaltend

I) 5 bis 80 Gew.-%, bevorzugt 20 bis 60 Gew.-%, besonders bevorzugt 30 bis 40 Gew.-% eines Copolymers, erhältlich durch radikalische Copolymerisation von

A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation gegebenenfalls in Gegenwart mindestens eines polymeren Additivs mit zahlenmittleren Molekulargewichten von 200 g/mol bis $10^9$ g/mol erfolgt,
H) mit der Maßgabe, dass die Komponente A) mit mindestens einer Komponente ausgewählt aus einer der Gruppen D) bis G) copolymerisiert wird,

II) 20 bis 95 Gew.-%, bevorzugt 30 bis 80 Gew.-%, besonders bevorzugt 40 bis 60 Gew.-% eines oder mehrerer Emulgatoren und/oder einer Ölphase und
III) 0 bis 30 Gew.-%, bevorzugt 0 bis 10 Gew.-%, besonders bevorzugt 0 bis 5 Gew.-% Wasser.

Die Copolymere gemäß Komponente I) besitzen bevorzugt ein Molekulargewicht von $10^3$ bis $10^9$ g/mol, besonders bevorzugt von $10^4$ bis $10^7$ g/mol, insbesondere bevorzugt $5*10^4$ bis $5*10^6$ g/mol.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die $Li^+$-, $Na^+$-, $K^+$-, $Mg^{++}$-, $Ca^{++}$-, $Al^{+++}$und/oder $NH_4^+$-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um ($C_1$ - $C_{22}$)-Alkylreste oder ($C_2$ - $C_{10}$)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind. Der Neutralisationsgrad der Acryloyldimethyltaurinsäure kann zwischen 0 und 100 % betragen, besonders bevorzugt ist ein Neutralisationsgrad von oberhalb 80 %.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.
Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.
Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind $Li^+$, $Na^+$, $K^+$, $Mg^{++}$, $Ca^{++}$, $Al^{+++}$, $NH_4^+$, Monoalkylammonium-, Dialkylammonium-, Trialkylammonium- und/oder Tetraalkylammoniumreste, wobei es sich bei den Alkylsubstituenten der Amine unabhängig voneinander um ($C_1$ - $C_{22}$)-Alkylreste oder ($C_2$- $C_{10}$)-Hydroxyalkylreste handeln kann. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100% betragen.
Als Comonomere B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acrylund Methacrylsäure, bevorzugt Acrylamid, Methacrylamid, N,N-Dimethyl-acrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat; Diethylaminomethylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen, zwitterionischen, oder amphoteren Struktur tragen.
Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphomeren Derivate überführt werden können.

Besonders bevorzugt als Comonomere C) sind Diallyldimethylammoniumchlorid (DADMAC), [2-(Methacryloyloxy)ethyl]trimethylammoniumchlorid (MAPTAC), [2-(Acryloyloxy)ethyl]trimethylammoniumchlorid, [2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder Methacryloylethyl-betain.

Der Gewichtsanteil der Comonomeren C) kann, bezogen auf die Gesamtmasse der Copolymere, 0,1 bis 99,8 Gew.-%, bevorzugt 0,5 bis 30 Gew.-% und besonders bevorzugt 1 bis 20 Gew.-% betragen.
Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte silikonhaltige Komponenten sind solche gemäß Formel (I).

$$R^1 — Z\text{-}[(Si(R^3R^4)\text{-}O\text{-})_w — (Si(R^5R^6)\text{-}O)_x — ] — R^2 \qquad (I)$$

Dabei stellt $R^1$ eine polymerisationsfähige Funktion aus der Gruppe der vinylisch ungesättigten Verbindungen dar, die zum Aufbau polymerer Strukturen auf radikalischem Wege geeignet ist. Bevorzugt stellt $R^1$ einen Vinyl-, Allyl-, Methallyl-, Methylvinyl-, Acryl- ($CH_2$=CH-CO-), Methacryl- ($CH_2$=C[$CH_3$]-CO-), Crotonyl-, Senecionyl-, Itaconyl-, Maleinyl-, Fumaryl- oder Styrylrest dar.
Zur Anbindung der silikonhaltigen Polymerkette an die reaktive Endgruppe $R^1$ ist eine geeignete chemische Brücke Z erforderlich. Bevorzugte Brücken Z sind -O-, -(($C_1$-$C_{50}$)Alkylen)-, -(($C_6$-$C_{30}$)Arylen)-, -(($C_5$-$C_8$)Cycloalkylen)-, -(($C_1$-$C_{50}$) Alkenylen)-, -(Polypropylenoxid)$_n$-, -(Polyethylenoxid)$_o$-, -(Polypropylenoxid)$_n$(Polyethylenoxid)$_o$-, wobei n und o unabhängig voneinander Zahlen von 0 bis 200 bedeuten und die Verteilung der EO/PO-Einheiten statistisch oder blockförmig sein kann. Weiterhin geeignet als Brückegruppierungen Z sind -(($C_1$ - $C_{10}$)Alkyl)-(Si($OCH_3$)$_2$)- und -(Si($OCH_3$)$_2$)-.
Der polymere Mittelteil wird durch silikonhaltige Wiederholungseinheiten repräsentiert.
Die Reste $R^3$, $R^4$, $R^5$ und $R^6$ bedeuten unabhängig voneinander -$CH_3$, -O-$CH_3$, -$C_6H_5$ oder -O-$C_6H_5$.
Die Indizes w und x repräsentieren stöchiometrische Koeffizienten, die unabhängig voneinander 0 bis 500, bevorzugt 10 bis 250, betragen.

Die Verteilung der Wiederholungseinheiten über die Kette hinweg kann nicht nur rein statistisch, sondern auch blockartig, alternierend oder gradientenartig sein kann.
$R^2$ kann einerseits einen aliphatischen, olefinischen, cycloaliphatischen, arylaliphatischen oder aromatischen ($C_1$ - $C_{50}$)-Kohlenwasserstoffrest symbolisieren (linear oder verzweigt) oder -OH, -$NH_2$, -N($CH_3$)$_2$, -$R^7$ oder für die Struktureinheit [-Z-$R^1$] stehen. Die Bedeutung der beiden Variablen Z und $R^1$ wurde bereits erklärt. $R^7$ steht für weitere Si-haltige Gruppierungen. Bevorzugte $R^7$-Reste sind -O-Si($CH_3$)$_3$, -O-Si(Ph)$_3$, -O-Si(O-Si($CH_3$)$_3$)$_2$$CH_3$) und -O-Si(O-Si (Ph)$_3$)$_2$Ph).
Wenn $R^2$ ein Element der Gruppe [-Z-$R^1$] darstellt, handelt es sich um difunktionelle, Monomere, die zur Vernetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylischoder methacrylisch modifizierten silikonhaltigen Komponenten:

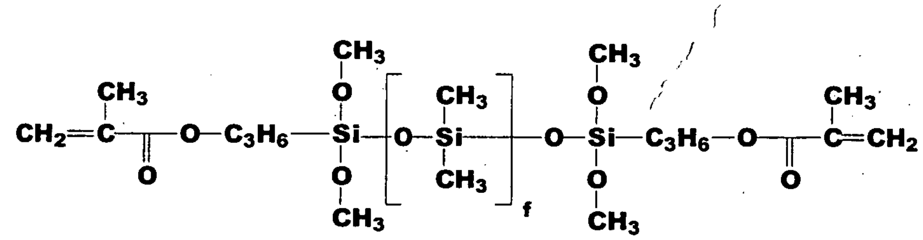

Methacryloxypropyldimethylsilyl endgeblockte Polydimethylsiloxane (f = 2 bis 500)

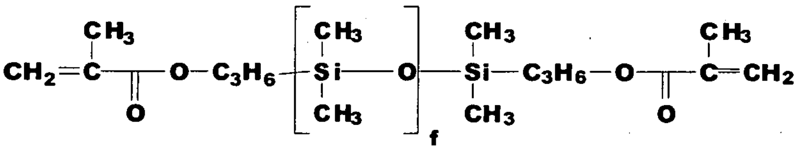

Methacryloxypropyl endgeblockte Polydimethylsiloxane (f = 2 bis 500)

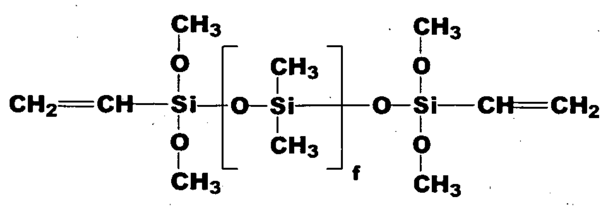

Vinyldimethoxysilyl endgeblockte Polydimethylsiloxane (f = 2 bis 500)

Bezogen auf die Gesamtmasse der Copolymere kann der Gehalt an siliziumhaltigen Komponenten bis 99,9 Gew.-%, bevorzugt 0,5 bis 30 Gew.-%, insbesondere bevorzugt 1 bis 20 Gew.-%, betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri-, oder polyfunktionelle Komponenten E) zu zumindest teilvernetzten Strukturen führen.

Bevorzugte fluorhaltige Komponenten E) sind solche gemäß Formel (II).

$$R^1\text{-}Y\text{-}C_rH_{2r}C_sF_{2s}CF_3 \qquad \text{(II)}$$

Dabei stellt $R^1$ eine polymerisationsfähige Funktion aus der Gruppe der vinylisch ungesättigten Verbindungen dar, die zum Aufbau polymerer Strukturen auf radikalischem Wege geeignet ist. Bevorzugt stellt $R^1$ ein Vinyl-, Allyl-, Methallyl-, Methylvinyl-, Acryl- ($CH_2$=CH-CO-), Methacryl- ($CH_2$=C[$CH_3$]-CO-), Crotonyl-, Senecionyl-, Itaconyl-, Maleinyl-, Fumaryl- oder Styrylrest, besonders bevorzugt einen Acryl- und Methacrylrest, dar.

Zur Anbindung der fluorhaltigen Gruppierung an die reaktive Endgruppe $R^1$ ist eine geeignete chemische Brücke Y erforderlich. Bevorzugte Brücken Y sind -O-, -C(O)-, -C(O)-O-, -S-, -O-$CH_2$-CH(O-)-$CH_2$OH, -O-$CH_2$-CH(OH)-$CH_2$-O-, -O-$SO_2$-O- , -O-S(O)-O-, -PH-, -P($CH_3$)-, -$PO_3$-, -NH-, -N($CH_3$)-, -O-($C_1$-$C_{50}$)Alkyl-O-, -O-Phenyl-O-, -O-Benzyl-O-, -O-($C_5$-$C_8$)Cycloalkyl-O-, -O-($C_1$-$C_{50}$)Alkenyl-O-, -O-(CH($CH_3$)-$CH_2$-O)$_n$-, -O-($CH_2$-$CH_2$-O)$_n$- und -O-([CH-$CH_2$-O]$_n$-[$CH_2$-$CH_2$-O]$_m$)$_o$-, wobei n, m und o unabhängig voneinander Zahlen von 0 bis 200 bedeuten und die Verteilung der EO- und PO-Einheiten statistisch oder blockförmig sein kann.

Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctylethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctylethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99,9

Gew.-%, bevorzugt 0,5 bis 30 Gew.-%, insbesondere bevorzugt 1 bis 20 Gew.-%, betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen, die aus einer oder mehreren Wiederholungseinheit(en) aufgebaut sind und eine für Polymere charakteristische Molekulargewichtsverteilung aufweisen.

Bevorzugt als Makromonomere F) sind Verbindungen gemäß Formel (III).

$$R^1 - Y - [(A)_v - (B)_w - (C)_x - (D)_z] - R^2 \qquad \text{(III)}$$

$R^1$ stellt eine polymerisationsfähige Funktion aus der Gruppe der vinylisch ungesättigten Verbindungen dar, die zum Aufbau polymerer Strukturen auf radikalischem Wege geeignet sind. Bevorzugt stellt $R^1$ einen Vinyl-, Allyl-, Methallyl-, Methylvinyl-, Acryl- ($CH_2$=CH-CO-), Methacryl- ($CH_2=C[CH_3]$-CO-), Crotonyl-, Senecionyl-, Itaconyl-, Maleinyl-, Fumaryl- oder Styrylrest dar.

Zur Anbindung der Polymerkette an die reaktive Endgruppe ist eine geeignete verbrückende Gruppe Y erforderlich. Bevorzugte Brücken Y sind -O-, -C(O)-, -C(O)-O-, -S-, -O-$CH_2$-CH(O-)-$CH_2$OH, -O-$CH_2$-CH(OH)-$CH_2$O-, -O-$SO_2$-O-, -O-$SO_2$-O-, -O-SO-O-, -PH-, -P($CH_3$)-, -$PO_3$-, -NH- und -N($CH_3$)-, besonders bevorzugt -O-.

Der polymere Mittelteil des Makromonomeren wird durch die diskreten Wiederholungseinheiten A, B, C und D repräsentiert. Bevorzugte Wiederholungseinheiten A,B,C und D leiten sich ab von Acrylamid, Methacrylamid, Ethylenoxid, Propylen-oxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.

Die Indizes v, w, x und z in Formel (III) repräsentieren die stöchiometrische Koeffizienten betreffend die Wiederholungseinheiten A, B, C und D. v, w, x, und z betragen unabhängig voneinander 0 bis 500, bevorzugt 1 bis 30, wobei die Summe der vier Koeffizienten im Mittel ≥1 sein muss.

Die Verteilung der Wiederholungseinheiten über die Makromonomerkette kann statistisch, blockartig, alternierend oder gradientenartig sein.

$R^2$ bedeutet einen linearen oder verzweigten aliphatischen, olefinischen, cycloaliphatischen, arylaliphatischen oder aromatischen ($C_1$-$C_{50}$)-Kohlenwasserstoffrest, OH, -$NH_2$, -N$(CH_3)_2$ oder ist gleich der Struktureinheit [-Y-$R^1$].

Im Falle von $R^2$ gleich [-Y-$R^1$] handelt es sich um difunktionelle Makromonomere, die zur Vernetzung der Copolymere geeignet sind.

Besonders bevorzugt als Makromonomere F) sind acrylisch- oder methacrylisch monofunktionalisierte Alkylethoxylate gemäß Formel (IV).

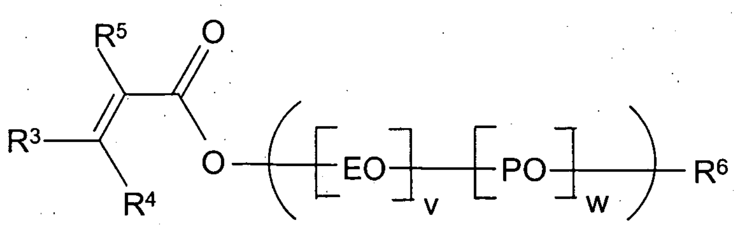

(IV)

$R_3$, $R_4$, $R_5$ und $R_6$ bedeuten unabhängig voneinander Wasserstoff oder n-aliphatische, iso-aliphatische, olefinische, cycloaliphatische, arylaliphatische oder aromatische ($C_1$-$C_{30}$)-Kohlenwasserstoffreste.

Bevorzugt sind $R_3$ und $R_4$ gleich H oder -$CH_3$, besonders bevorzugt H; $R_5$ ist gleich H oder -$CH_3$; und $R_6$ ist gleich einem n-aliphatischen, iso-aliphatischen, olefinischen, cycloaliphatischen, arylaliphatischen oder aromatischen ($C_1$-$C_{30}$)-Kohlenwasserstoffrest.

v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥1 sein muss. Die Verteilung der EO- und PO-Einheiten über die Makromonomerkette kann statistisch, blockartig, alternierend oder gradientenartig sein.

Weiterhin insbesondere bevorzugte Makromonomeren F) haben die folgende Struktur gemäß Formel (IV):

| Bezeichnung | $R^3$ | $R^4$ | $R^5$ | $R^6$ | v | w |
|---|---|---|---|---|---|---|
| Genapol ®LA-030-methacrylat | H | H | -$CH_3$ | -Lauryl | 3 | 0 |

(fortgesetzt)

| Bezeichnung | $R^3$ | $R^4$ | $R^5$ | $R^6$ | v | w |
|---|---|---|---|---|---|---|
| Genapol ®LA-070-methacrylat | H | H | $-CH_3$ | -Lauryl | 7 | 0 |
| Genapol ®LA-200-methacrylat | H | H | $-CH_3$ | -Lauryl | 20 | 0 |
| Genapol ®LA-250-methacrylat | H | H | $-CH_3$ | -Lauryl | 25 | 0 |
| Genapol ®T-080-methacrylat | H | H | $-CH_3$ | -Talk | 8 | 0 |
| Genapol ®T-080-acrylat | H | H | H | -Talk | 8 | 0 |
| Genapol ®T-250-methacrylat | H | H | $-CH_3$ | -Talk | 25 | 0 |
| Genapol ®T-250-crotonat | $-CH_3$ | H | $-CH_3$ | -Talk | 25 | 0 |
| Genapol ®OC-030-methacrylat | H | H | $-CH_3$ | -Octyl | 3 | 0 |
| Bezeichnung | $R^3$ | $R^4$ | $R^5$ | $R^6$ | v | w |
| Genapol ®OC-105-methacrylat | H | H | $-CH_3$ | -Octyl | 10 | 5 |
| Genapol ®Behenyl-010-metharyl | H | H | H | -Behenyl | 10 | 0 |
| Genapol ®Behenyl-020-metharyl | H | H | H | -Behenyl | 20 | 0 |
| Genapol ®Behenyl-010-senecionyl | $-CH_3$ | $-CH_3$ | H | -Behenyl | 10 | 0 |
| Genapol ®PEG-440-diacrylat | H | H | H | -Acryl | 10 | 0 |
| Genapol ®B-11-50-methacrylat | H | H | $-CH_3$ | -Butyl | 17 | 13 |
| Genapol ®MPEG-750-methacrylat | H | H | $-CH_3$ | -Methyl | 18 | 0 |
| Genapol ®P-010-acrylat | H | H | H | -Phenyl | 10 | 0 |
| Genapol ®O-050-acrylat | H | H | H | -Oleyl | 5 | 0 |

Weiterhin als Makromonomere F) insbesondere geeignet sind Ester der (Meth)acrylsäure mit ($C_{10}$-$C_{18}$)-Fettalkoholpolyglykolethern mit 8 EO-Einheiten (Genapol® C-080) $C_{11}$-Oxoalkoholpolyglykolethern mit 8 EO-Einheiten (Genapol® UD-080) ($C_{12}$-$C_{14}$)-Fettalkoholpolyglykolethern mit 7 EO-Einheiten (Genapol® LA-070) ($C_{12}$-$C_{14}$)-Fettalkoholpolyglykolethern mit 11 EO-Einheiten (Genapol® LA-110) ($C_{16}$-$C_{18}$)-Fettalkoholpolyglykolethern mit 8 EO-Einheiten (Genapol® T-080) ($C_{16}$-$C_{18}$)-Fettalkoholpolyglykolethern mit 15 EO-Einheiten (Genapol® T-150) ($C_{16}$-$C_{18}$)-Fettalkoholpolyglykolethern mit 11 EO-Einheiten (Genapol® T-110) ($C_{16}$-$C_{18}$)-Fettalkoholpolyglycolethern mit 20 EO-Einheiten (Genapol® T-200) ($C_{16}$-$C_{18}$)-Fettalkoholpolyglycolethern mit 25 EO-Einheiten (Genapol® T-250) ($C_{18}$-$C_{22}$)-Fettalkoholpolyglycolethern mit 25 EO-Einheiten und/oder iso-($C_{16}$-$C_{18}$)-Fettalkoholpolyglycolethern mit 25 EO-Einheiten Bei den Genapol®-Typen handelt es sich um Produkte der Firma Clariant, GmbH.

Bevorzugt beträgt das Molekulargewicht der Makromonomeren F) 200 g/mol bis $10^6$ g/mol, besonders bevorzugt 150 bis $10^4$ g/mol und insbesondere bevorzugt 200 bis 5000 g/mol.

Bezogen auf die Gesamtmasse der Copolymere können geeignete Makromonomere bis zu 99,9 Gew.-% eingesetzt werden. Bevorzugt finden die Bereiche 0,5 bis 30 Gew.-% und 70 bis 99,5 Gew.-% Anwendung. Besonders bevorzugt sind Anteile von 1 bis 20 Gew.-% und 75 bis 95 Gew.-%.

Bevorzugt als Copolymere sind solche, die durch Copolymerisation mindestens der Komponenten A), C) und D) erhältlich sind.

Weiterhin bevorzugt als Copolymere sind solche, die durch Copolymerisation mindestens der Komponenten A), C) und E) erhältlich sind.

Weiterhin bevorzugt als Copolymere sind solche, die durch Copolymerisation mindestens der Komponenten A), D) und F) erhältlich sind.

Weiterhin bevorzugt als Copolymere sind solche, die durch Copolymerisation mindestens der Komponenten A) und F) erhältlich sind.

In einer bevorzugten Ausführungsform wird die Copolymerisation in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.

Die Additive G) bzw. deren Mischungen müssen lediglich ganz oder teilweise im gewählten Polymerisationsmedium

löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropf-Copolymerisation statistisch von aktiven Radikalen angegriffen. Dies führt dazu, dass je nach Additiv G) mehr oder weniger große Anteile davon in die Copolymere eingebaut werden. Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen.
Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole.
Insbesondere bevorzugt als Additive G) sind Polyvinylpyrrolidone (z.B. Luviskol K15®, K20® und K30® von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive G) beträgt bevorzugt $10^2$ bis $10^7$ g/mol, besonders bevorzugt $0{,}5*10^4$ bis $10^6$ g/mol.

Die Einsatzmenge des polymeren Additivs G) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.
Insbesondere bevorzugt als Vernetzer ist Trimethylolpropantriacrylat (TMPTA).
Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodiisobutyronitril (AIBN), verwendet werden.
Ebenfalls geeignet sind anorganische Peroxyverbindungen, wie z.B. $(NH_4)_2S_2O_8$,
$K_2S_2O_8$ oder $H_2O_2$, gegebenenfalls in Kombination mit Reduktionsmitteln (z.B. Natriumhydrogensulfit, Ascorbinsäure, Eisen(II)-sulfat etc.) oder Redoxsystemen, welche als reduzierende- Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser/Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).
Bevorzugt eignet sich die Fällungspolymerisation, besonders bevorzugt die Fällungspolymerisation in tert.-Butanol.

Die nachfolgende Auflistung zeigt 67 Copolymere, die für die Formulierung der erfindungsgemäßen Mittel besonders geeignet sind. Die verschiedenen Copolymere Nr. 1 bis Nr. 67 sind gemäß den folgenden Herstellverfahren 1, 2, 3 und 4 erhältlich.

Verfahren 1:

Diese Polymere sind nach dem Fällungsverfahren in tert.-Butanol herstellbar. Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere wurden nach beendeter Reaktion (2 Stunden) durch Absaugen des Lösungsmittels und durch anschließende Vakuumtrocknung isoliert.

Verfahren 2:

Diese Polymere sind nach dem Gelpolymerisationsverfahren in Wasser herstellbar. Dabei wurden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt $Na_2S_2O_8$) gestartet. Die Polymergele wurden anschließend zerkleinert und nach Trocknung die Polymere isoliert.

Verfahren 3:

Diese Polymere sind nach dem Emulsionsverfahren in Wasser herstellbar. Dabei wurden die Monomere in einer Mischung aus Wasser/organ. Lösungsmittel (bevorzugt Cyclohexan) unter Verwendung eines Emulgators emulgiert, die Reaktionsmischung mittels $N_2$ inertisiert und anschließend die Reaktion nach-Anheizen auf 80°C durch Zugabe von geeigneten Initiatoren- oder Initiatorsystemen (bevorzugt $Na_2S_2O_8$) gestartet. Die Polymeremulsionen wurden anschließend eingedampft (Cyclohexan fungiert als Schlepper für Wasser) und dadurch die Polymere isoliert.

Verfahren 4:

Diese Polymere sind nach dem Lösungsverfahren in organischen Lösungsmitteln (bevorzugt Toluol, z.B. auch tert. Alkohole) herstellbar. Dabei wurden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere wurden durch Abdampfen des Lösungsmittels und durch anschließende Vakuumtrocknung isoliert.

| Polymere mit hydrophoben Seitenketten, unvernetzt | | | |
|---|---|---|---|
| Nr. | Zusammensetzung | | Herstellverfahren |
| 1 | 95 g AMPS | 5 g Genapol T-080-methacrylat | 1 |
| 2 | 90 g AMPS | 10 g Genapol T-080-methacrylat | 1 |
| 3 | 85 g AMPS | 15 g Genapol T-080-methacrylat | 1 |
| 4 | 80 g AMPS | 20 g Genapol T-080-methacrylat | 1 |
| 5 | 70 g AMPS | 30 g Genapol T-080-methacrylat | 1 |
| 6 | 50 g AMPS | 50 g Genapol T-080-methacrylat | 3 |
| 7 | 40 g AMPS | 60 g Genapol T-080-methacrylat | 3 |
| 8 | 30 g AMPS | 70 g Genapol T-080-methacrylat | 3 |
| 9 | 20 g AMPS | 80 g Genapol T-080-methacrylat | 3 |
| 10 | 60 g AMPS | 60 g BB10-acrylat | 4 |
| 11 | 80 g AMPS | 20 g BB10-acrylat | 4 |

(fortgesetzt)

| Polymere mit hydrophoben Seitenketten, unvernetzt | | |
|---|---|---|
| Nr. | Zusammensetzung | Herstellverfahren |
| 12 | 90 g AMPS 10 g BB10-methacrylat | 3 |
| 13 | 80 g AMPS 20 g BB10-methacrylat | 1 |
| 14 | 80 g AMPS 20 g Genapol LA040-acrylat | 1 |

| Polymere mit hydrophoben Seitenketten, vernetzt | | |
|---|---|---|
| Nr. | Zusammensetzung | Herstellverfahren |
| 15 | 80 g AMPS 20 g Genapol LA040-methacrylat 0,6 g AMA | 1 |
| 16 | 80 g AMPS 20 g-Genapol LA040-methacrylat 0,8 g AMA | 1 |
| 17 | 80 g AMPS 20 g Genapol LA040-methacrylat 1,0 g AMA | 1 |
| 18 | 628,73 g AMPS 120,45 g Genapol T-250-acrylat 6,5 g TMPTA | 2 |
| 19 | 60 g AMPS 40 g BB10-acrylat 1,9 g TMPTA | 4 |
| 20 | 80 g AMPS 20 g BB10-acrylat 1,4 g TMPTA | 4 |
| 21 | 90 g AMPS 10 g BB10-methacrylat 1,9 g TMPTA | 4 |
| 22 | 80 g AMPS 20 g BB25-methacrylat 1,9 g TMPTA | 1 |
| 23 | 60 g AMPS 40 g BB10-acrylat 1,4 g TMPTA | 4 |

| Polymere mit hydrophoben Seitenketten, vernetzt, gepfropft | | |
|---|---|---|
| Nr. | Zusammensetzung | Herstellverfahren |
| 24 | 95 g AMPS 5 g BB10-acrylat, 1,9 g TMPTA, 1 g Poly-NVP | 1 |
| 25 | 90 g AMPS 10 g BB10-acrylat, 1,9 g TMPTA, 1 g Poly-NVP | 1 |
| 26 | 85 g AMPS 15 g BB10-acrylat, 1,9 g TMPTA, 1 g Poly-NVP | 1 |
| 27 | 90 g AMPS 10 g BB10-methacrylat, 1,9 g TMPTA, 1 g Poly-NVP | 1 |

| Polymere mit siliziumhaltigen Gruppen, unvernetzt | | |
|---|---|---|
| Nr. | Zusammensetzung | Herstellverfahren |
| 28 | 80 g AMPS, 20 g Silvet 867 | 1 |
| 29 | 80 g AMPS, 50 g Silvet 867 | 4 |

| Polymere mit siliziumhaltigen Gruppen, vernetzt | | |
|---|---|---|
| Nr. | Zusammensetzung | Herstellverfahren |
| 30 | 80 g AMPS, 20 g Silvet 867, 0,5 g MBA | 4 |
| 31 | 80 g AMPS, 20 g Silvet 867, 1,0 g MBA | 1 |
| 32 | 60 g AMPS, 40 g Y-12867, 0,95 g AMA | 1 |
| 33 | 80 g AMPS, 20 g Y-12867, 0,95 g AMA | 1 |
| 34 | 90 g AMPS, 10 g Y-12867, 0,95 g AMA | 1 |

(fortgesetzt)

| Polymere mit siliziumhaltigen Gruppen, vernetzt | | |
|---|---|---|
| Nr. | Zusammensetzung | Herstellverfahren |
| 35 | 60 g AMPS, 40 g Silvet 7280, 0,95 g AMA | 1 |
| 36 | 80 g AMPS, 20 g Silvet 7280, 0,95 g AMA | 1 |
| 37 | 90 g AMPS, 10 g Silvet 7280, 0,95 g AMA | 1 |
| 38 | 60 g AMPS, 40 g Silvet 7608, 0,95 g AMA | 1 |
| 39 | 80 g AMPS, 20 g Silvet 7608, 0,95 g AMA | 1 |
| 40 | 90 g AMPS, 10 g Silvet 7608, 0,95 g AMA | 1 |

| Polymere mit hydrophoben Seitenketten und kationischen Gruppen, unvernetzt | | |
|---|---|---|
| Nr. | Zusammensetzung | Herstellverfahren |
| 41 | 87,5 g AMPS, 7,5 g Genapol T-110, 5 g DADMAC | 2 |
| 42 | 40 g AMPS, 10 g Genapol T110, 45 g Methacrylamid | 2 |
| 43 | 55 g AMPS, 40 g Genapol LA040, 5 g Quat | 1 |
| 44 | 75 g AMPS, 10 g BB10, 6,7 g Quat | 1 |

| Polymere mit hydrophoben Seitenketten und kationischen Gruppen, vernetzt | | |
|---|---|---|
| Nr. | Zusammensetzung | Herstellverfahren |
| 45 | 60 g AMPS, 20 g Genapol T-80, 10 g Quat, 10 g HEMA | 1 |
| 46 | 75 g AMPS, 20 g GenapolT-250, 5 g Quat, 1,4 g TMPTA | 1 |
| 47 | 75 g AMPS, 20 g GenapolT-250, 10 g Quat, 1,4 g TMPTA | 1 |
| 48 | 75 g AMPS, 20 g GenapolT-250, 20 g Quat, 1,4 g TMPTA | 1 |

| Polymere mit fluorhaltigen Gruppen | | |
|---|---|---|
| Nr. | Zusammensetzung | Herstellverfahren |
| 49 | 94 g AMPS, 2,02 g Fluowet AC 600 | 1 |
| 50 | 80 g AMPS, 20 g Perfluoroctylpolyethylenglykolmethacrylat, 1 g Span 80 | 3 |

| Polymere mit fluorhaltigen Gruppen, gepfropft | | |
|---|---|---|
| Nr. | Zusammensetzung | Herstellverfahren |
| 51 | 80 g AMPS, 10 g Fluowet AC 600, 5 g Poly-NVP | 1 |
| 52 | 70 g AMPS, 8 g Perfluoroctylethyloxyglycerinmethacrylat, 5 g Poly-NVP | 4 |

| Multifunktionelle Polymere | | |
|---|---|---|
| Nr. | Zusammensetzung | Herstellverfahren |
| 53 | 80 g AMPS, 10 g Genapol LA070, 10 g Silvet 7608, 1,8 g TMPTA | 1 |

(fortgesetzt)

| Multifunktionelle Polymere | | |
|---|---|---|
| Nr. | Zusammensetzung | Herstellverfahren |
| 54 | 70 g AMPS, 5 g N-Vinylpyrrolidon, 15 g Genapol T-250 methacrylat, 10 g Quat, 10 g Poly-NVP | 4 |
| 55 | 80 g AMPS, 5 g N-Vinylformamid, 5 g Genapol O-150-methacrylat, 10 g DADMAC, 1,8 g TMPTA, 8 g Poly-N-Vinylformamid | 2 |
| 56 | 70 g AMPS, 5 g N-Vinylpyrrolidon, 15 g Genapol T-250-methacrylat, 10 g Quat, 10 g Poly-NVP | 1 |
| 57 | 60 g AMPS, 10 g Genapol-BE-020-methacrylat, 10 g Genapol T-250-acrylat, 20 g Quat, 1 g Span 80 | 1 |
| 58 | 60 g AMPS, 20 g MPEG-750-methacrylat, 10 g Methacryloxypropyldimethicon, 10 g Perfluorooctylpolyethylenglycol-methacrylat, 10 g Poly[N-vinylcaprolacton-co-acrylsäure] (10/90) | 1 |
| 59 | 80 g AMPS, 5 g N-Vinylformamid, 5 g Genapol O-150-methacrylat, 10 g DADMAC, 1,8 g TMPTA | 1 |
| 60 | 70 g AMPS, 10 g Genapol T-250-acrylat, 5 g N-Methyl-4-vinylpyridiniumchlorid, 2,5 g Silvet Y-12867, 2,5 g Perfluorhexylpolyethylenglykolmethacrylat, 10 g Polyethylenglykoldimethacrylat, 4 g Poly[N-Vinylcaprolactam] | 1 |
| 61 | 10 g AMPS, 20 g Acrylamid, 30 g N-2-Vinylpyrrolidon, 20 g Silvet 7608, 10 g Methacryloxypropyl dimethicon, 10 g Fluowet AC 812 | 3 |
| 62 | 60 g AMPS, 10 g DADMAC, 10 g Quat, 10 g Genapol-LA-250-crotonat, 10 g Methacryloxypropyldimethicon, 7 g Poly[acrylsäure-co-N-vinylformamid] | 1 |
| 63 | 50 g AMPS, 45 g Silvet 7608, 1,8 g TMPTA, 8 g Poly[N-Vinylformamid] | 1 |
| 64 | 20 g AMPS, 10 g Genapol T 110, 35 g MAA, 30 g HEMA, 5 g DADMAC | 4 |
| 65 | 20 g AMPS, 80 g BB10, 1,4 g TMPTA | 1 |
| 66 | 75 g AMPS, 20 g BB10, 6,7 g Quat, 1,4 g TMPTA | 1 |
| 67 | 35 g AMPS, 60 g Acrylamid, 2 g VIFA, 2,5 g Vinylphosphonsäure, 2 Mol-% Fluowet EA-600 | 4 |

Chemische Bezeichnung der Reaktanden:

| | |
|---|---|
| AMPS | Acryloyldimethyltaurat, wahlweise Na- oder $NH_4$-Salz |
| Genapol® T-080 | $C_{16}$-$C_{18}$-Fettalkoholpolyglykolether mit 8 EO-Einheiten |
| Genapol® T-110 | $C_{12}$-$C_{14}$-Fettalkoholpolyglykolether mit 11 EO-Einheiten |
| Genapol® T-250 | $C_{16}$-$C_{18}$-Fettalkoholpolyglycolether mit 25 EO-Einheiten |
| Genapol® LA-040 | $C_{12}$-$C_{14}$-Fettalkoholpolyglykolether mit 4 EO-Einheiten |
| Genapol® LA-070 | $C_{12}$-$C_{14}$-Fettalkoholpolyglykolether mit 7 EO-Einheiten |
| Genapol® O-150 methacrylat | $C_{16}$-$C_{18}$-Fettalkoholpolyglykolether methacrylat mit 15 EO-Einheiten, |
| Genapol® LA-250 crotonat | $C_{12}$-$C_{14}$-Fettalkoholpolyglykolether crotonat mit 25 EO-Einheiten |
| Genapol® T-250 methacrylat | $C_{16}$-$C_{18}$-Fettalkoholpolyglycolether methacrylat mit 25 EO-Einheiten |
| Genapol® T-250 acrylat | $C_{16}$-$C_{18}$-Fettalkoholpolyglycolether methacrylat mit 25 EO-Einheiten |
| BB10® | Polyoxyethylen(10)Behenylether |
| TMPTA | Trimethylolpropantriacrylat |
| Poly-NVP | Poly-N-Vinylpyrrolidon |
| Silvet® 867 | Siloxan Polyalkylenoxid Copolymer |
| MBA | Methylen-bis-acrylamid |
| AMA | Allylmethacrylat |

| | |
|---|---|
| ®Y-12867 | Siloxan Polyalkylenoxid Copolymer |
| Silvet® 7608 | Polyalkylenoxid-modifiziertes Heptamethyltrisiloxan |
| Silvet® 7280 | Polyalkylenoxid-modifiziertes Heptamethyltrisiloxan |
| DADMAC | Dialiyldimethyl-ammoniumchlorid |
| HEMA | 2-Hydroxyethylmethacrylat |
| Quat | 2-(Methacryloyloxy)ethyltrimethylammoniumchlorid |
| Fluowet® AC 600 | Perfluoralkylethylacrylat |
| Span® 80 | Sorbitanester |

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden.

Die beschriebene, optional durchführbare Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in rinse off Produkten (insbesondere Haarbehandlungsmittel) als auch leave on Produkten (insbesondere O/W Emulsionen) gewünscht sein.

Die erfindungsgemäßen Dispersionskonzentrate enthalten neben dem Copolymer noch einen oder mehrere Emulgatoren und/oder eine Ölphase in der angegebenen Menge. Bei Verwendung von Emulgatoren als alleiniger Komponente II ist der Anteil der Ölphase somit 0 % und entsprechend ist der Anteil der Emulgatoren 0 %, wenn die Komponente II nur aus einer Ölphase besteht. Bevorzugt verwendet man als zweite Komponente eine Mischung aus Emulgator und Ölphase.

Als Emulgatoren kommen in Betracht Anlagerungsprodukte von 0 bis 30 Mol Alkylenoxid, insbesondere Ethylen-, Propylen-, Butylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitanester; ($C_{12}$-$C_{18}$)-Fettsäuremonound -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Bevorzugt sind flüssige Fettsäureester, die sowohl ethoxyliert (PEG-10 Polyglyceryl-2 Laurate) als auch nicht ethoxyliert (Polyglyceryl-2 Sesquiisostearate) sein können.

Weitere erfindungsgemäße Dispersionskonzentrate enthalten bevorzugt Sorbitolester, hergestellt durch Reaktion von Sorbitol mit Fettsäuremethylestern oder Fettsäuretriglyceriden entsprechend dem in DE 197 27 950 beschriebenen Verfahren. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rapsöl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern. Die erfindungsgemäß eingesetzten Sorbitolester können auch alkoxyliert sein, vorzugsweise ethoxyliert.

Des weiteren können anionische Emulgatoren, wie ethoxylierte und nicht ethoxylierte mono-, di- oder tri-Phosphorsäureester, aber auch kationische Emulgatoren wie mono-, di- und tri-Alkylquats und deren polymere Derivate eingesetzt werden.

Ebenfalls geeignet sind Gemische von Verbindungen aus mehreren dieser Substanzklassen.

Die erfindungsgemäßen Dispersionen können neben AMPS-Copolymer ein oder mehrere Öle enthalten, bevorzugt aus der Gruppe der Kohlenwasserstoffe, Esteröle, pflanzlichen Öle und Silikonöle.

Die erfindungsgemäß eingesetzten Öle umfassen Kohlenwasserstofföle mit linearen oder verzweigten, gesättigten oder ungesättigten C7-C40-Kohlenstoffketten, beispielsweise Vaseline, Dodecan, Isododecan, Cholesterol, Lanolin, hydrierte Polyisobutylene, Docosane, Hexadecan, Isohexadecan, Paraffine und Isoparaffine;

Öle pflanzlichen Ursprungs, insbesondere flüssige Triglyceride wie Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl;

Öle tierischen Ursprungs, beispielweise Rindertalg, Schweineschmalz, Gänseschmalz, Perhydrosqualen, Lanolin; synthetische Öle wie Purcellinöl, lineare und/oder verzweigte Fettalkohole und Fettsäureester, bevorzugt Guerbetalkohole mit 6 bis 18, vorzugsweise 8 bis 10, Kohlenstoffatomen; Ester von linearen ($C_6$-$C_{13}$)-Fettsäuren mit linearen ($C_6$-$C_{20}$)-Fettalkoholen; Ester von verzweigten ($C_6$-$C_{13}$)-Carbonsäuren mit linearen ($C_6$-$C_{20}$)-Fettalkoholen, Ester von linearen ($C_6$-$C_{18}$)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von $C_1$-$C_{10}$-Carbonsäuren oder $C_2$-$C_{30}$-Dicarbonsäuren, $C_1$-$C_{30}$-Carbonsäuremonoester und Polyester von Zucker, $C_1$-$C_{30}$-Monoester und Polyester von Glycerin;
Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle.

Monoglyceride von $C_1$-$C_{30}$-Carbonsäuren, Diglyceride von $C_1$-$C_{30}$-Carbonsäuren, Triglyceride von $C_1$-$C_{30}$-Carbonsäuren, beispielsweise Triglyceride der Capryl/Caprinsäuren, Ethylenglykolmonoester von $C_1$-$C_{30}$-Carbonsäuren, Ethylenglycoldiester von $C_1$-$C_{30}$-Carbonsäuren, Propylenglycolmonoester von $C_1$-$C_{30}$-Carbonsäuren, Propylenglycoldiester von $C_1$-$C_{30}$-Carbonsäuren, sowie propoxilierte und ethoxilierte Derivate der oben genannten Verbindungsklassen.

An Silikonölen in Betracht kommen Dimethylpolysiloxane, Cyclomethicone, Polydialkylsiloxane $R_3SiO(R_2SiO)_xSiR_3$, wobei R für eine Methyl und Ethyl, besonders bevorzugt für Methyl steht und x für eine Zahl von 2 bis 500 steht, beispielsweise unter den Handelsnamen VICASIL (General Electric Company), DOW CORNING 200, DOW CORNING 225, DOW CORNING 200 (Dow Corning Corporation), erhältlichen Dimethicone. Trimethylsiloxysilicate $[(CH_2)_3SiO)1/2]_x[SiO_2]_y$, wobei x für eine Zahl von 1 bis 500 und y für eine Zahl von 1 bis 500 steht. Dimethiconole $R_3SiO[R_2SiO]_xSiR_2OH$ und $HOR_2SiO[R_2SiO]_xSiR_2OH$, wobei R für Methyl oder Ethyl und x für eine Zahl bis zu 500 steht, Polyalkylarylsiloxane, beispielsweise unter den Handelsbezeichnungen SF 1075 METHYLPHENYL FLUID (General Electric Company) und 556 COSMETIC GRADE PHENYL TRIMETHICONE FLUID (Dow Corning Corporation) erhältliche Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere, wie in US 5104 645 und den darin zitierten Schriften beschrieben, die bei Raumtemperatur sowohl flüssig als auch harzförmig vorliegen können.

Die Herstellung der erfindungsgemäßen Dispersionskonzentrate kann auf verschiedene Weisen erfolgen, wobei eine inverse Emulsionspolymerisation oder eine inverse Mini-Emulsionspolymerisation genauso bevorzugt ist wie eine physikalische Abmischung von AMPS-Copolymer mit Öl-/ Emulgator- und ggfs. Wasserphase. Die physikalische Abmischung erfolgt bevorzugt in der Weise, dass Ölphase und Emulgator/en bei 10 bis 60°C, bevorzugt bei Raumtemperatur vermischt werden, zu ca. 40 Gew.-% der Öl-/Emulgatorphase wird dann AMPS-Copolymer/e in einem Zeitraum von 10 bis 60 Min, bevorzugt ca. 30 Min. unter kräftigem Rühren zugegeben. Dabei bildet sich eine homogene Paste. Wenn erforderlich, kann eine geringe Menge an Wasser zum besseren Verarbeiten hinzugefügt werden. Anschließend wird die restliche Öl-/Emulgatorphase unter Rühren zugegeben und mehrere Stunden homogen verrührt. Es resultiert eine flüssige, gießfähige Dispersion.

Die erfindungsgemäßen Dispersionskonzentrate eignen sich als Verdicker, Konsistenzgeber, Emulgator, Solubilisator, Dispergator, Gleitmittel, Haftmittel, Conditioner und/oder Stabilisator - hervorragend zur Formulierung von kosmetischen, pharmazeutischen und dermatologischen Mitteln, insbesondere von Öl-in-Wasser Emulsionen in Form von Cremes, Lotionen, Reinigungsmilch, Cremegele, Sprühemulsionen, z.B. Bodylotions, After-Sun Lotions, Sonnenschutzmittel und Deo-Sprays.

Die erfindungsgemäßen Dispersionskonzentraten werden in den kosmetischen und pharmazeutischen Zubereitungen in Gewichtsmengen eingesetzt, dass Polymerkonzentrationen von 0,01 bis 10 Gew.-%, bevorzugt 0,1 bis 5 Gew.-%, besonders bevorzugt 0,5 bis 3 Gew.-%, bezogen auf die fertigen Mittel resultieren.

Die erfindungsgemäßen Mittel können anionische, kationische, nichtionische, zwitter-ionische und/oder amphotere Tenside, sowie weitere Hilfs- und Zusatzstoffe, kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.

Zusätzlich können die erfindungsgemäßen Mittel organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin

(Glycerintriacetat) und 1-Methoxy-2-propanol. Hydrotrop wirken kurzkettige Aniontenside, insbesondere Arylsulfonate, beispielsweise Cumol- oder Toluolsulfonat.

Die nachfolgenden Beispiele sollen den Gegenstand der Erfindung näher erläutern, ohne ihn darauf einzuschränken (bei den Prozentangaben handelt es sich um Gew.-%).

Es wurden verschiedene Basisrezepturen mit hoher Emulgatorkonzentration (A1 bis A6) sowie mit niedriger Emulgatorkonzentration (B1 bis B6) hergestellt. Dabei wurden jeweils verschiedene Polymere eingesetzt:
Polymer Nr. 1, 2, 4, 17, 18, 22, 27, 28, 32, 41, 44 sowie 46 gemäß den obigen Tabellen. Die resultierenden Polymerdispersionen wurden nach Aussehen, Viskosität sowie Stabilität (Sedimentierung bei Lagerung 25°C 3 Wochen) beurteilt.

| 1. Hohe Emulgatorkonz. | | | | | | |
|---|---|---|---|---|---|---|
| | Polymer Nr. | | | | | |
| | 18 | 22 | 17 | 41 | 22 | 22 |
| | A1 | A2 | A3 | A4 | A5 | A6 |
| Polymer | 36,0 % | 36,0 % | 36,0 % | 36,0 % | 36,0 % | 36 % |
| Hostacerin DGI | 25,6 % | 12,8 % | 51,2 % | 25,6 % | 28,8 % | 30 % |
| Hostaphat KL 340 D | 6,4 % | 19,2 % | 12,8 % | 38,4 % | 19,2 % | 18 % |
| Myritol 318 | 32,0 % | 32,0 % | 0,0% | 16,0 % | 24,0 % | 16 % |
| Paraffin | 0,0 % | 0,0 % | 0,0 % | 0,0 % | 0,0 % | 0,0 % |
| IPP | 0,0% | 0,0 % | 0,0 % | 0,0 % | 0,0 % | 0,0 % |

| 1. Niedrige Emulgatorkonz. | | | | | | |
|---|---|---|---|---|---|---|
| | Polymer Nr. | | | | | |
| | 1 | 4 | 44 | 18 | 22 | 22 |
| | B1 | B2 | B3 | B4 | B5 | B6 |
| Polymer | 36,0 % | 36,0% | 36,0 % | 36,0 % | 36,0 % | 36 % |
| Hostacerin DGI | 4,0 % | 2,0 % | 4,0 % | 2,0 % | 3,0 % | 3 % |
| Hostaphat KL 340 D | 1,0 % | 3,0 % | 1,0 % | 3,0 % | 2,0 % | 2 % |
| Myritol 318 | 59,0 % | 59,0 % | 0,0 % | 0,0 % | 29,5 % | 0,0% |
| Paraffin | 0,0 % | 0,0 % | 59,0 % | 59,0 % | 29,5 % | 29,5% |
| IPP | 0,0 % | 0,0 % | 0,0 % | 0,0 % | 0,0 % | 29,5% |

A 2-6 mischen und ein Drittel der Lösung vorlegen.
B In einer halben Stunde das HMP-Polymer unter Rühren bei 400 upm zugeben.
C Eine halbe Stunde nachrühren dann die restliche Lösung von A zugeben.
D Weitere 5 Stunden nachrühren.

Chemische Bezeichnung der eingesetzten Handelsprodukte:

| | |
|---|---|
| Hostacerin DGI | Polyglyceryl-2 Sesquiisostearate |
| Myritol 318 | Caprylic/Capric Triglyceride |
| IPP | Isopropylpalmitat |
| Hostaphat KL340D | Trilaureth-4-Phosphat |

**Patentansprüche**

**1.** Dispersionskonzentrate, enthaltend:

I) 5 bis 80 Gew.-%, bevorzugt 20 bis 60 Gew.-%, besonders bevorzugt 30 bis 40 Gew.-% eines Copolymers, erhältlich durch radikalische Copolymerisation von

A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation gegebenenfalls in Gegenwart mindestens eines polymeren Additivs mit zahlenmittleren Molekulargewichten von 200 g/mol bis $10^9$ g/mol erfolgt,
H) mit der Maßgabe, dass die Komponente A) mit mindestens einer Komponente ausgewählt aus einer der Gruppen D) bis G) copolymerisiert wird,

II) 20 bis 95 Gew.-%, bevorzugt 30 bis 80 Gew.-%, besonders bevorzugt 40 bis 60 Gew.-% eines oder mehrerer Emulgatoren und/oder einer Ölphase und
III) 0 bis 30 Gew.-%, bevorzugt 0 bis 10 Gew.-%, besonders bevorzugt 0 bis 5 Gew.-% Wasser.

**2.** Dispersionskonzentrate nach Anspruch 1, **dadurch gekennzeichnet, dass** sie ein Copolymer enthalten, das 20 bis 99,5 Gew.-%, bezogen auf die Gesamtmasse des Copolymers, Acryloyldimethyltaurinsäure oder deren Salz enthält.

**3.** Dispersionskonzentrat nach Anspruch 1, **dadurch gekennzeichnet, dass** es 20 bis 60 Gew.-% des Copolymers enthält.

**4.** Dispersionskonzentrat nach Anspruch 1, **dadurch gekennzeichnet, dass** es 30 bis 40 Gew.-% des Copolymers enthält.

**5.** Dispersionskonzentrat nach Anspruch 1, **dadurch gekennzeichnet, dass** es 30 bis 80 Gew.-% Emulgator und/oder Ölphase enthält.

**6.** Dispersionskonzentrat nach Anspruch 1, **dadurch gekennzeichnet, dass** es 40 bis 60 Gew.-% Emulgator und/oder Ölphase enthält.

**7.** Dispersionskonzentrat nach Anspruch 1, **dadurch gekennzeichnet, dass** es 0 bis 10 Gew.-% Wasser enthält.

**8.** Dispersionskonzentrat nach Anspruch 1, **dadurch gekennzeichnet, dass** es 0 bis 5 Gew.-% Wasser enthält.

**9.** Kosmetische, pharmazeutische oder dermatologische Zubereitungen, enthaltend ein Dispersionskonzentrat nach Anspruch 1.

**10.** Kosmetische, pharmazeutische oder dermatologische Zubereitungen in Form von Öl-in-Wasser Emulsionen, enthaltend ein Dispersionskonzentrat nach Anspruch 1.